# EUROPEAN PATENT APPLICATION

(11) **EP 0 638 279 A1**
(43) Date of publication of application: **15.02.1995**
(21) Application number: 94302679.9
(22) Date of filing: 14.04.1994
(51) Int. Cl.: A61B 1/00, A61B 17/32

(54) **Endoscope with retractable tool**

(30) Priority: 06.08.1993 US 102736
(71) Applicant: CITATION MEDICAL CORPORATION, Reno, Nevada 89502 (US)
(72) Inventor: Middle, George H., Reno, Nevada 89509 (US); Evans, Edward, Sparks, Nevada 89436 (US); Purdy, Craig, Sparks, Nevada 89436 (US); Freiman, Allen, Reno, Nevada 89506 (US)
(74) Representative: Coxon, Philip

(57) **Abstract**

An endoscope has a fiber optic scope assembly and a probe (12), through which illuminating optic fibers (30) pass, along with an image guide. The probe also contains a surgery tool (34) which can be selectively extended from the outer end of the probe or retracted into the probe. The probe can also contain passageways (46,47) for the flow of irrigating fluid into or out of the treatment area.

## Description

This is a continuation-in-part patent application of co-pending U.S. Patent Application Serial No. 08/093368, filed on July 16, 1993, and entitled "Endoscope with Integral Tool."

### FIELD OF THE INVENTION

The present invention relates to medical diagnostic and treatment devices. More particularly, the present invention relates to hand held endoscopes used to examine and treat an internal area of a patient.

### BACKGROUND OF THE INVENTION

Historically, diagnosis and treatment of an internal organ or a bone joint of a patient often required the performance of significantly invasive surgery on the patient. More recently, techniques and equipment have been developed which allow the internal examination of these areas through the use of endoscopy, a much less invasive procedure. The endoscope includes a slender probe which contains an imaging device. The imaging device is connected through the probe to an eyepiece or a video display screen. Endoscopy involves the insertion of the probe into the joint or other area to be examined, followed by examination of the area either through an eyepiece or on a video display screen.

It is also now possible to perform relatively non-invasive surgery by insertion of a slender cutting tool through a separate incision into the area to be treated, followed by performance of the necessary surgery with the cutting tool. The surgery is viewed in progress by the physician through an endoscope as described above for the examination procedure. Viewing can be either through an eyepiece or on a video display screen.

This procedure requires two entry points into the area to be treated, one for the endoscope, and one for the cutting tool. Even though the entry incisions are relatively small, they result in some residual soreness of the area, and they constitute some risk of infection, either from the instrument or from post-operative contamination. Additionally, the insertion of multiple instruments into a joint or other area, and the subsequent manipulation of those instruments will result in some internal trauma to the patient, which is unavoidable.

Further, the insertion and manipulation of multiple instruments can be awkward for the physician, and it may be difficult to maneuver the endoscope to effectively view the operations being performed by the cutting tool. This awkwardness is especially detrimental if the surgery tool being used has a cutting contour that is always exposed, subjecting the surrounding tissue to possible inadvertent lacerations. Finally, endoscopes and cutting tools that are not designed to be disposable must be sterilized before use, so the use of multiple instruments means an increased burden of sterilization.

Accordingly, it is an object of the present invention to provide an endoscope which is capable of performing a variety of surgical operations with a retractable tool, as well as providing a means of examination of the area during surgery. It is a further object of the present invention to provide an endoscope which contains means for illuminating a treatment area, means for the viewing the area, and means for performing the surgical treatment, all on one instrument. It is a still further object of the present invention to provide an endoscope which is relatively inexpensive to manufacture and comparatively easy and cost-effective to use.

### SUMMARY OF THE INVENTION

In a preferred embodiment which is only exemplary of the present invention, a portable endoscope has a hand held scope assembly, with an elongated probe attached to the scope assembly. The probe includes a tubular steel needle which is mounted on a generally cylindrical handle assembly. The needle has a plurality of passages therethrough, leading to a plurality of openings in its distal end, to facilitate illumination, viewing, and treatment of the area to be treated. A surgery tool is installed in one of the passages so as to be extended through one of the openings in the distal end of the scope needle, or retracted within the opening. The endoscope can be inserted directly through an incision into the treatment area of the patient, or it can be inserted through a hollow tubular cannula which has been inserted into the incision.

For illumination of the treatment area, a plurality of optical illumination fibers are permanently mounted within one of the passages in the scope needle, extending from the opening in the distal end down through the needle to the scope at the proximal end of the instrument. The proximal ends of these fibers are illuminated with light from a suitable light source in the scope, such as a quartz halogen lamp. The location of the light source could also be external to the instrument, with the light being transmitted to the illumination fibers by a suitable optical cable. The light from this source is transmitted by the illumination fibers to the opening in the distal end of the scope needle, where it illuminates the area to be examined and treated.

A cylindrical gradient refractive index (GRIN) rod is also permanently mounted within the distal end of the needle, at an opening in the end, alongside the illumination fibers. Light reflected by the interior structure of the area to be treated enters the distal end of the cylindrical GRIN rod. The image formed by this reflected light is focussed by the GRIN rod onto the proximal end of the cylindrical GRIN rod.

For transfer of this image back to the scope, the proximal end of the GRIN rod is attached to the distal end of a fiber optic image guide. This image guide is permanently mounted within one of the passages in the scope needle, and it extends from the GRIN rod back through the needle to the scope at the proximal end. The proximal end of the fiber optic image guide is optically joined to focussing optics in an eyepiece or mounted on an attached camera assembly. These focussing optics are axially movable for focussing the image either for viewing directly on an eyepiece or for video display by an attached camera.

In addition, installed within one of the passages through the scope needle is a surgery or cutting tool, which can have a variety of shapes, as desired. The operative contours of the tool can be straight, curved, pointed or blunt, as required for the surgery to be performed. If the endoscope is used in conjunction with a cannula, it can be withdrawn and replaced with another instrument having a tool with another configuration, with relative ease. The portion of the surgery tool having the cutting contour can be selectively extended from the opening at the distal end of the blade passageway, or retracted into the opening, as desired.

Therefore, illuminating light is shed by a light source, received by the proximal end of the illuminating optical fibers, and transmitted by those optical fibers to the open distal end of the scope needle. At the distal end of the needle, which has been inserted into the patient, at the treatment area, the illuminating light exits the needle. Portions of this light are reflected by the internal structure of the treatment area, and the reflected light is received by the distal end of the GRIN rod. The GRIN rod focusses the reflected light onto its proximal end as the image of the internal structure of the treatment area.

This image is then transmitted by the optical fiber image guide to focussing optics where the image is focussed for viewing either through an eyepiece or on a video display tube. While viewing the image of the internal structure of the treatment area, the physician can examine the area, determine the proper treatment, and extend the surgery tool through the distal end of the scope needle to perform any required surgery. The surgery tool can also be withdrawn as desired, to facilitate further manipulation of the instrument without causing unwanted trauma to surrounding tissue.

The novel features of this invention, as well as the invention itself, both as to its structure and its operation, will be best understood from the accompanying drawings, taken in conjunction with the accompanying description, in which similar reference characters refer to similar parts, and in which:

### BRIEF DESCRIPTION OF THE DRAWINGS

Fig. 1 is a perspective view of the endoscope of the present invention in its intended environment;
Fig. 2 is a perspective view of a preferred embodiment of the endoscope of the present invention, showing the major components;
Fig. 3 is a partial section view of the distal end of the probe of the endoscope shown in Fig. 2;
Fig. 4 is a section view of the distal end of the probe of the endoscope shown in Fig. 2, with the surgery tool extended;
Fig. 5 is a section view of the distal end of the probe of the endoscope shown in Fig. 2, with the surgery tool retracted;
Fig. 6 is a perspective view of a preferred embodiment of the positioning button of the endoscope shown in Fig. 2;
Fig. 7 is a section view of the endoscope shown in Fig. 2, showing an exemplary ratchet mechanism;
Fig. 8 is a section view of the endoscope shown in Fig. 2, showing an alternative embodiment of the ratchet mechanism; and
Figs. 9 - 13 are perspective views of the distal end of the probe of the endoscope shown in Fig. 2, showing examples of different types of surgery tools which can be used.

### DESCRIPTION OF PREFERRED EMBODIMENTS

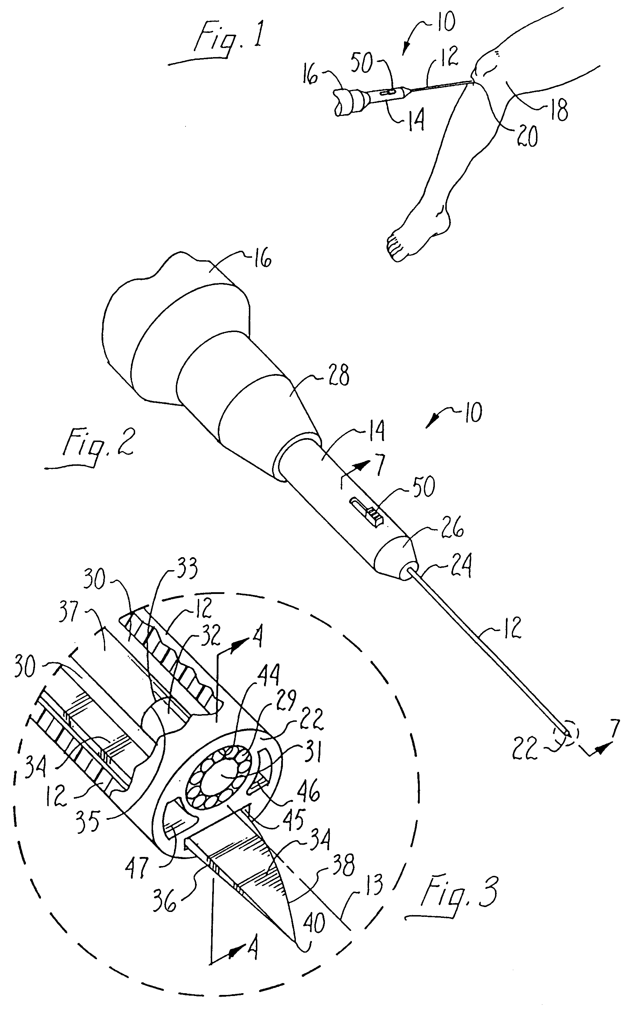

As seen in Fig. 1, the endoscope 10 includes a scope needle probe 12, which is attached to a generally cylindrical handle 14, to which is also attached a scope assembly 16. The physician inserts the needle probe 12 into an incision 20 in the knee 18 of a patient. The endoscope 10 can be used to examine and treat various body parts, and it can have various configurations of these basic components according to the application. It is shown here for treatment of a knee, simply by way of example. Instead of being inserted directly into an incision 20, as is known in the art, it is also known in the art that it can be inserted through a cannula (not shown) which has been inserted into the incision 20.

As seen in Fig. 2, on a larger scale, endoscope 10 has an elongated, slender, hollow tubular probe 12, which has a distal end 22 and a proximal end 24. Proximal end 24 of probe 12 is fixedly attached to distal end 26 of handle 14. Scope interface 28 is attached directly to handle 14, and scope assembly 16 is attached to handle 14 via scope interface 28. Scope interface 28, as is known in the art, serves to transfer illumination light from the scope assembly 16 to components in handle 14, and to transfer an optical image of the treatment area from handle 14 to scope assembly 16 for display. Surgery tool positioning button 50 is located on handle 14, for retracting or extending a surgery tool from probe 12, as will be described later. During use, the physician handles the instrument by handle 14, distal end 22 of probe 12 is inserted into the treatment area of the patient, and the image of the treatment area can be observed through an eyepiece or a video display connected at scope assembly 16. If surgery is required, positioning button 50 can be moved forward to extend a surgery tool from the distal end 22 of probe 12.

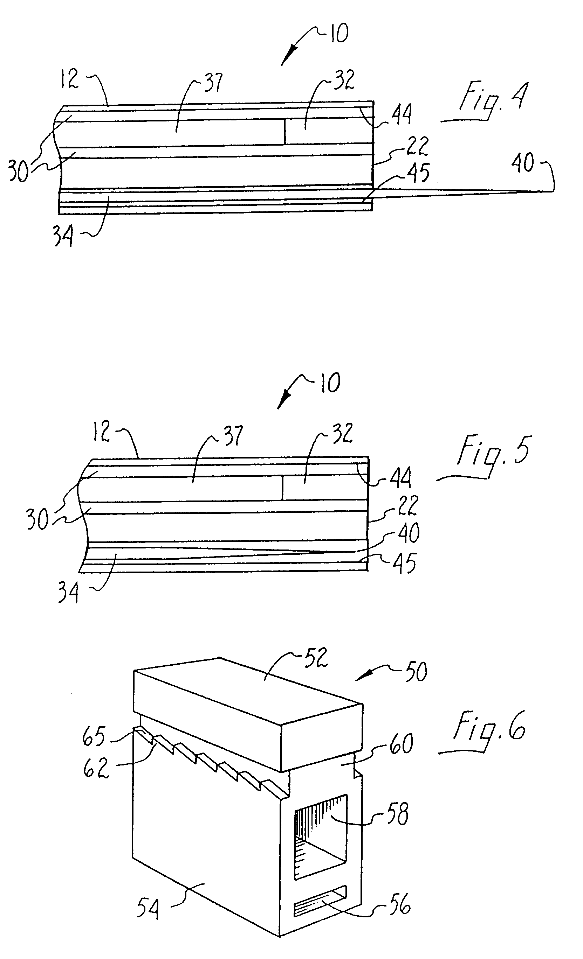

Fig. 3 shows an even larger view of a portion of endoscope 10. Probe 12 is shown to be a tubular needle which has a plurality of longitudinal internal passageways 44, 45, 46, and 47, and which has corresponding openings at its distal end 22. Illumination optical fibers 30 run the full length of probe 12, generally parallel to the longitudinal axis 13 of probe 12. The number, size, and arrangement of illumination fibers 30 can vary from one to any number, as long as they transmit sufficient light to their distal ends 29 to illuminate the area to be examined and treated. As shown in Fig. 3, illumination fibers 30 are arranged around the inside diameter of passageway 44 of probe 12. The method of permanently mounting illumination fibers 30 inside probe 12 in this arrangement is known in the art and does not constitute part of the present invention. Similarly, illumination fibers 30 are connected as is known in the art to a light source via the proximal end 24 of probe 12.

Also shown in Fig. 3 is the distal end 31 of GRIN rod 32, which is permanently installed parallel to the longitudinal axis 13 of probe 12. Again, GRIN rod 32, or some other light gathering lens structure could be in a variety of sizes, shapes and configurations without departing from the present invention, as long as it gathers sufficient light and focusses the image sharply enough to provide a clear image for viewing by the physician. As is known in the art, the proximal end 33 of GRIN rod 32 is attached to the distal end 35 of an image guide in the form of an optical fiber 37, which then extends the full length of probe 12. Optical fiber 37 is connected at its proximal end (not shown) in a conventional way for the transmission of an image to a viewing device via scope assembly 16.

Still in Fig. 3, protruding through distal end 22 of probe 12 are fluid passageways 46 and 47, which run the length of probe 12. Passageway 46 can be provided for the introduction of irrigation fluid into the treatment area to remove blood or tissue from the area being viewed. Passageway 47 can be provided for the aspiration of tissue or fluid from the treatment area. These passageways in probe 12 can be connected to the appropriate fluid ducts (not shown) for the transport of fluid through the handle 14 and beyond, by means that are well known in the art.

According to the present invention, also shown in Fig. 3 is a surgery tool 34 mounted in passageway 45 so as to be extendable beyond, or retractable within, the distal end 22 of probe 12. Surgery tool 34 extends generally parallel with the longitudinal axis 13 of probe 12. The particular blade or surgery tool 34 shown in Fig. 3 is a relatively flat blade with a sharp tip 40 on its distal end. It also has a blunt edge 36 on one side and a sharp, curved cutting edge 38 on the opposite side, both oriented substantially in line with the periphery of probe 12. The embodiment of surgery tool 34 shown in Fig. 3 is intended only as an example, and it can have a variety of shapes, sizes, and orientations, without departing from the present invention.

As best seen in Figs. 4 and 5, surgery tool 34 can be extended from passageway 45 a sufficient distance to expose cutting edge 38, or surgery tool 34 can be retracted within passageway 45 to completely enclose cutting edge 38 and point 40. Passageway 45 is shown substantially in line with the periphery of probe 12, but it could be oriented radially, or in some other fashion, depending upon the type of surgery tool being used. The extended position of surgery tool 34, shown in Fig. 4, is used for implementation of the tool, and the retracted position, shown in Fig. 5, is used whenever the tool is not in use.

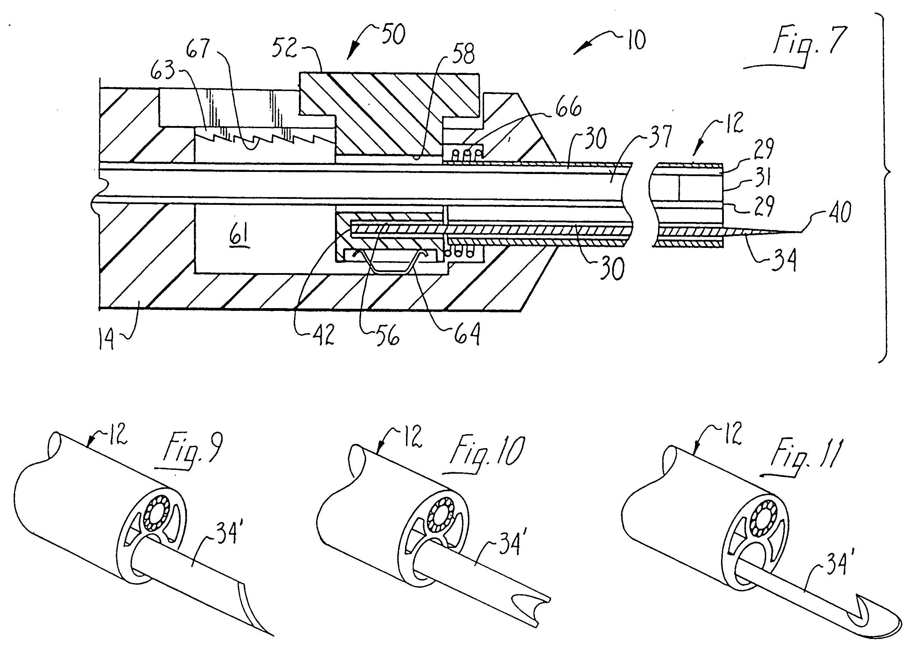

Figs. 6 and 7 show an example of the way in which surgery tool 34 can be extended and retracted. Several mechanisms for accomplishing this function are well known in the art, and the one shown here is only intended to illustrate the desired modes of operation of the chosen retraction mechanism. In summary, the tool 34 should be capable of extension to completely expose its useful contours, it should be capable of complete retraction to prevent its contours from contacting any surrounding tissue, and it should be latchable in the desired position. It would also be desirable to provide a number of selectable latched positions between the fully extended position and the fully retracted position.

Extension and retraction of tool 34 is accomplished by manipulation of positioning button 50, the functional details of which are shown in Fig. 6. Surgery tool 34 is attached to positioning button 50 by having surgery tool proximal end 42 inserted into socket 56, where it can be retained by any retention means known in the art. Port 58 is provided through button 50, to allow the free passage of illumination fibers 30, image guide optical fiber 37, and any fluid ducts, without interfering with the operation of positioning button 50. Ratchet mechanism 62 is shown as a series of ramps formed into side 54 of button 50 to provide an adjustable latch mechanism as will be described later. Upper surface 52 is the primary surface manipulated by the physician in extending and retracting the surgery tool 34.

As seen in Fig.7, positioning button 50 is installed in slot 61, which is recessed longitudinally in handle 14. Ratchet mechanism 62 on side 54 of positioning button 50 is preferably formed on both sides of button 50, and it meshes with similar ratchet mechanism 63 on the sides of slot 61 in handle 14, so that upper surface 65 of ratchet mechanism 62 mates with lower surface 67 of ratchet mechanism 63. Sides 54 on button 50 abut slot 61, to guide the movement of button 50 generally longitudinally along handle 14. Front surface 60 of button 50 contacts button return spring 66, so that as button 50 moves forwardly, toward the distal end 26 of handle 14, spring 66 is compressed between front surface 60 of button 50 and the forward end of slot 61. Spring 66 continually urges button 50 away from the forward end of slot 61, or rearwardly. Button latch spring 64 is compressed between the bottom of button 50 and the bottom of slot 61, continually urging button 50 upwardly, causing ratchet mechanism 62 to mesh with ratchet mechanism 63.

As can be seen, downward pressure on button 50 will further compress button latch spring 64 and disengage ratchet mechanisms 62 and 63. Then, forward pressure on button 50 will cause button 50 to move forwardly along slot 61 to the position desired, accompanied by forward movement of tool 34 along passageway 45. This results in the extension of surgery tool 34 out of probe 12, to the extent desired. Conversely, downward pressure on button 50, accompanied by a slackening of forward pressure, will disengage ratchet mechanisms 62 and 63, allowing spring 66 to move button 50 rearwardly along slot 61 to the position desired. This results in the retraction of surgery tool 34 into probe 12, to the extent desired.

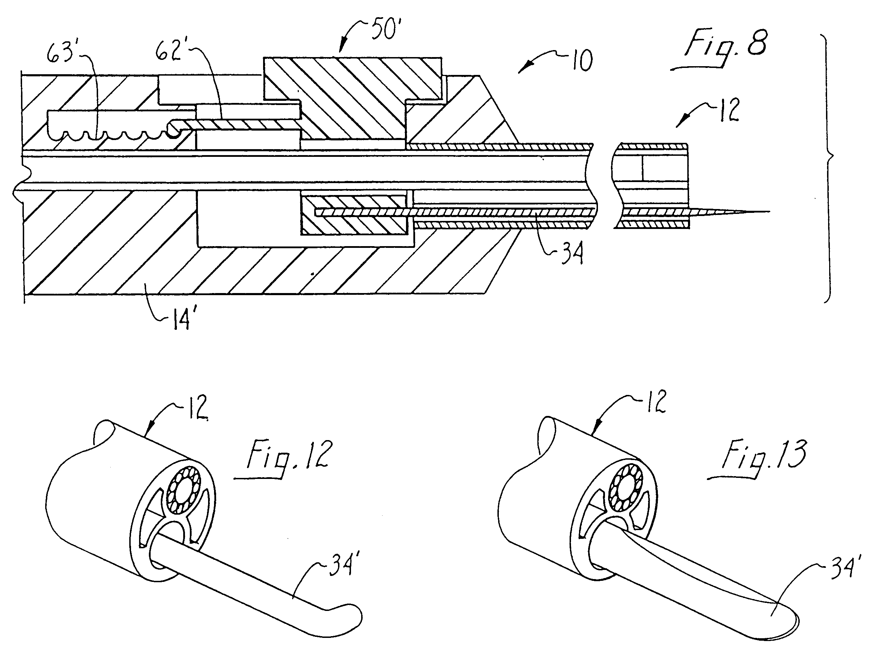

Fig. 8 shows another example of a way in which the surgery tool can be extended and retracted. Positioning button 50' slides longitudinally in a slot in handle 14' as described above. Ratchet mechanism 62' is a spring arm extending from positioning button 50' and meshing with ratchet mechanism 63', which is a series of detents formed internally in handle 14'. Surgery tool 34 is attached to positioning button 50' as described above. Moving positioning button 50' longitudinally along handle 14' will extend or retract tool 34 as desired.

As seen in Figs. 9 through 13, the endoscope of the present invention can incorporate a variety of surgery tools, examples of which are shown and designated 34'.

### OPERATION

The endoscope 10 of the present invention is inserted into incision 20 in the knee 18 of the patient, either directly or through a separate cannula. During insertion of the probe 12, surgery tool 34 is preferably retracted within probe 12. When the distal end 22 of the probe 12 is in the proximity of the area to be examined and treated, the physician can view the area to examine the pertinent tissue and formulate a treatment. If surgery is indicated, the physician can press forwardly on positioning button 50 to extend cutting tool 34 from probe 12 by the desired amount. Then, handle 14 can be manipulated to simultaneously maneuver the field of view and surgery tool 34 to cut, probe, or move tissue as required. Since surgery tool 34 will always be positioned in or near the illuminated field of view, the physician can view the surgery in progress with a minimum of awkwardness, and without having to simultaneously maneuver a multiplicity of instruments.

If a different surgery tool is required, endoscope 10 can be withdrawn from the incision and replaced with an endoscope having the desired instrument. When surgery is complete, or when maneuvering of the field of view is desired, surgery tool 34 can be retracted within probe 12.

While the particular endoscope with an integral retractable tool as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A hand held endoscope for examination and treatment of an internal structure of a patient, comprising:
a rigid probe having a proximal end and a distal end;
illuminating means fixedly mounted within said probe, for illumination of the internal structure of the patient;
viewing means fixedly mounted on said probe, for viewing the internal structure of the patient;
treatment means slidably mounted within said probe for treatment of the internal structure of the patient, said treatment means being slidable relative to said probe to selectively extend a portion of said treatment means from an opening on said distal end of said probe; and
manipulating means attached to said probe, for manipulation of said probe.

2. A hand held endoscope according to claim 1, wherein said illuminating means comprises:
a first optical fiber fixedly mounted within said probe and extending through said probe and terminating at said distal end of said probe; and
a light source attached to said endoscope so as to illuminate said first optical fiber.

3. A hand held endoscope according to claim 1 or 2, wherein said viewing means comprises:
a gradient refractive index rod fixedly mounted within said distal end of said probe, for gathering light reflected from the internal structure to be examined; and
a second optical fiber attached to said rod and extending from said rod through said proximal end of said probe.

4. A hand held endoscope according to any one of the preceding claims wherein:
said treatment means is a surgical knife; and
said portion of said treatment means, which can extend from said distal end of said probe, is a blade on said surgical knife.

5. A hand held endoscope according to claim 4, further comprising positioning means for positioning said blade on said surgical knife at a selected longitudinal position with respect to said probe to extend a desired portion of said blade beyond said distal end of said probe.

6. A hand held endoscope according to claim 5, wherein said selected longitudinal position of said blade can be chosen at a plurality of intermediate positions between a fully extended position and a fully retracted position.

7. A hand held endoscope according to claim 5 or 6, wherein said positioning means comprises:
a positioning button movably mounted on said manipulating means and attached to said surgical knife so as to move said blade to said selected longitudinal position with respect to said probe; and
a locking mechanism mounted on said manipulating means and connected to said positioning button so as to lock said blade at said selected longitudinal position.

8. A hand held endoscope according to claim 7, wherein:
said positioning button is slidably mounted on said manipulating means; and
said locking mechanism is a ratchet mechanism attached to said positioning button.

9. A hand held endoscope for examination and treatment of an internal structure of a patient, comprising:
a rigid, elongated probe having a proximal end and a distal end;
a plurality of passageways extending longitudinally through said probe from said proximal end to said distal end;
a light source attached to said endoscope;
an illuminating optical fiber mounted within a first of said passageways to transmit light from said light source to said distal end of said probe to illuminate the internal structure of the patient;
a gradient refractive index rod fixedly mounted at said distal end of said probe within said first passageway to gather light reflected from the internal structure of the patient;
a viewing optical fiber attached to said rod and mounted within said first passageway to transmit light from said rod to said proximal end of said probe for viewing;
a surgical knife, having a proximal end and a distal end, slidably mounted within a second of said passageways;
a blade on said distal end of said knife; and
a positioning button slidably mounted on said endoscope and attached to said proximal end of said surgical knife to move said surgical knife to a selected longitudinal position with respect to said probe to extend a desired portion of said blade beyond said distal end of said probe.

10. An endoscope according to claim 9, further comprising a plurality of illuminating optical fibers mounted within said first passageway through said probe.

11. An endoscope according to claim 9 or 10, wherein a fluid flows in a third of said passageways through said probe, for irrigation of the internal structure of the patient.

12. An endoscope according to claim 11, wherein:
said fluid flows into the patient through said third passageway; and
said fluid flows out of the patient through a fourth of said passageway through said probe.
